# EUROPEAN PATENT APPLICATION

(11) **EP 2 541 449 A1**
(43) Date of publication of application: **02.01.2013**
(21) Application number: 11172234.4
(22) Date of filing: 30.06.2011
(51) Int. Cl.: G06F 19/00

(54) **Physiological condition, diet and exercise plan recommendation and management system**

(71) Applicant: Wang, Leao, Taiping City, Taichung Hsien, 411 Taiwan R.O.C. (TW)
(72) Inventor: Wang, Leao, Taiping City, Taichung Hsien, 411 Taiwan R.O.C. (TW)
(74) Representative: 2K Patentanwälte Blasberg Kewitz & Reichel

(57) **Abstract**

The present invention discloses a physiological condition, diet and exercise plan recommendation and management system comprising a portable electronic device for recording a user's personal basic physiological parameters anytime, and drafting a copy of recommended food list according to the energy consumption and intake food list after exercise to remind users always concerning their food intake and exercise quantity in order to maintain good exercise habits and physiological conditions.

## Description

### BACKGROUND OF THE INVENTION

### 1. Fields of the Invention

The present invention relates to a physiological condition, diet and exercise plan recommendation and management system, and more particularly to a system capable of providing references for diet selection and recommendations for exercise to users anytime based on the relative relations among diet, physical activity and personal physiological changes.

### 2. Description of the Related Art

People have long been aware of the relative relation and the importance of diet, exercise and physiological changes (including weight, heart rate, blood pressure, body fat, etc.), but often fail to restrain their appetite and maintain good exercise habits due to both impacts of the temptation of delicious food and the laziness of doing exercise, and thus gradually ending up with a poor physiological condition or even a situation that requires medical assistance, before they aware of the need of controlling the diet and maintaining good exercise habits.

However, when a user wants to control the diet and build up the exercise habits, there is no related accessory equipment or instrument to help, remind or record the change of physiological parameters and the physical activities of the user, or provide an intake food list and an exercise recommendation to the user as references. Therefore, the user may take food at improper time or blindly do exercises according to the user's physiological changes (such as an increase of body weight) and try to restore the ideal physiological condition.

Just as most people have experienced, the diet control and the voluntary exercise mode usually give an effect which is not as good or even very frustrated and cannot be sustained. After all, the unorganized ways of controlling the diet and doing exercises compulsorily are similar to searching in dark and cannot improve the user's confidence and will, but more likely leading to a poor performance at the early stage and a doubt on the necessities of sustaining the exercise and maintaining the food selection. As a result, we may give up the plan easily and end up losing our health.

### SUMMARY OF THE INVENTION

Therefore, it is a primary objective of the present invention to use a general portable electronic device (such as a Smartphone, a PDA, a notebook, a tablet PC, an e-Book or any personal mobile device) to create a food list and allow a user to record all of the personal related physiological parameters, food intake conditions (and converting the food into calories) and exercise records anytime, as well as updating the recommended food list anytime as a reference for the user's diet.

Another objective of the present invention is to provide a commended exercise program, not only enhancing the exercising effect, but also offering an exclusive exercise recommendation in compliance with the user's need.

To achieve the foregoing objectives, the invention uses a portable electronic device to record the user's basic physiological parameters anytime and drafting a copy of recommended food list according to the energy consumption and the intake food list after the exercise to remind users to concern their food intake and exercise quantity and maintain good exercise habits and physiological conditions.

### BRIEF DESCRIPTION OF THE DRAWINGS

The accomplishment of this and other objects of the invention will become apparent from the following description and its accompanying drawings of which:
Fig. 1 is a flow chart of a basic control operation in accordance with one embodiment of the invention; and
Fig. 2 is a flow chart of a basic control operation in accordance with another embodiment of the invention.

### DETAILED DESCRIPTION OF THE PREFERRED EMBODIMENT

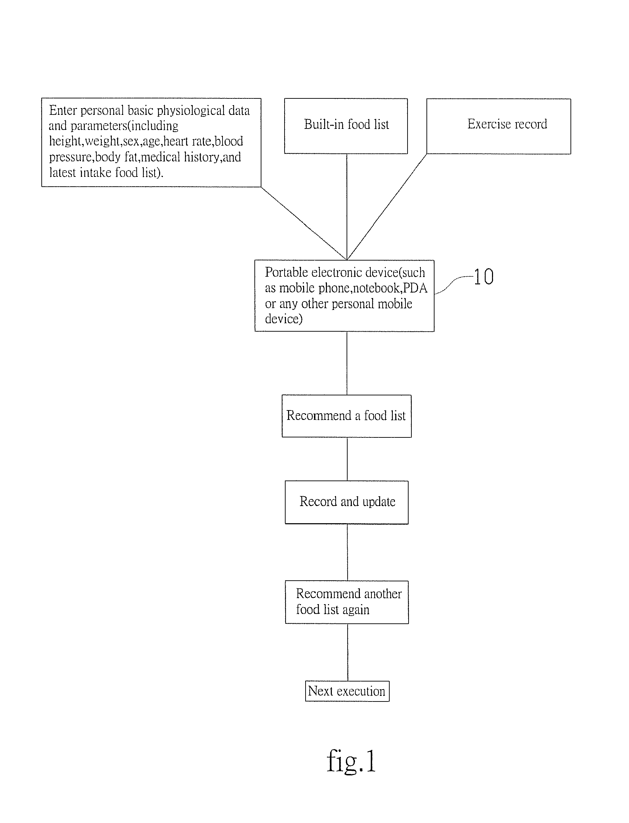

The technical measures and characteristics of the present invention will become apparent with the detailed description of preferred embodiments accompanied with related drawings as follows:
With reference to Fig. 1 for a flow chart of a basic control operation in accordance with one embodiment of the invention, executable software (not shown in figure) is installed to a portable electronic device 10, and a food list is built in the software, and the software of the portable electronic device 10 is provided for a user to enter personal basic information, intake food list and exercise record, and a network support of cable and wireless technologies (such as Bluetooth and Wi-Fi) is provided for entering the user's personal information including latest food intake, exercise, medication, professional medical advice, medical history, heart rate, blood pressure, weight, body fat, and sleep condition anytime, and the built-in software provides a recommended food list suitable for the user based on all of the parameters.

In other words, if the user's physiological conditions are normal and the user's exercise quantity is sufficient, then the recommended food list generated by the software will allow more choices for food (or having less restrains on diet). On the other hand, if the user's physiological conditions tend to be abnormal (such as overweight, and overeating) or the user's exercise quantity is insufficient, then the choices for food listed in the recommended food list will be reduced (or having more restrains on diet) and the management system reminds the user about diet and sustained exercise in order to maintain the best physiological condition and figure.

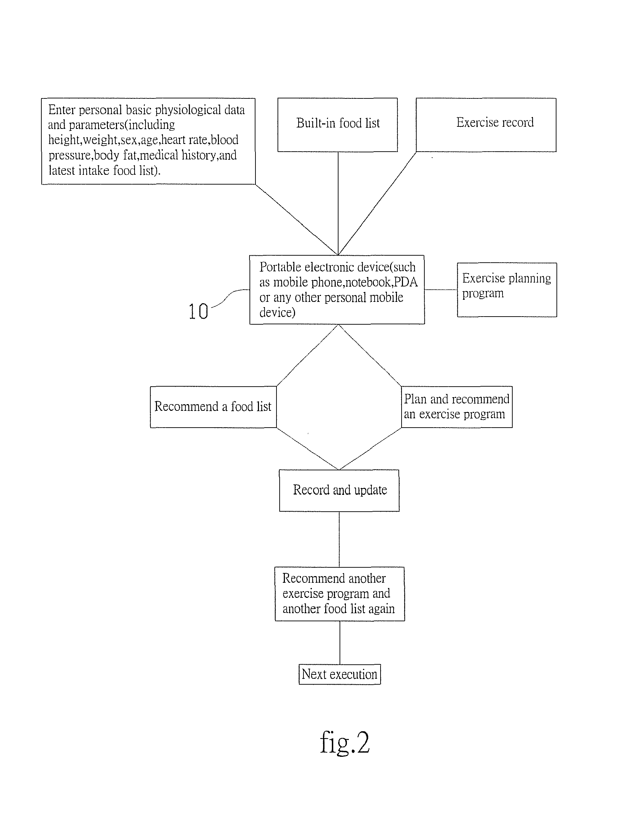

With reference to Fig. 2 for a flow chart of a basic control operation in accordance with another embodiment of the invention, an exercise planning program is installed to the original control and management system, so that after the software receives the necessary information such as the user's personal latest physiological data, intake food list and exercise record, a copy of recommended food list and a copy of recommended exercise program are listed, so that the user can carry out the best recommendation on diet and exercise according to the aforementioned two copies of lists.

Many changes and modifications in the above-described embodiments of the invention can, of course, be carried out without departing from the scope thereof. Accordingly, to promote the progress in science and the useful arts, the invention is disclosed and is intended to be limited only by the scope of the appended claims.

## Claims

1. A physiological condition, diet and exercise plan recommendation and management system, comprising
an executable software installed in a portable electronic device, and having a food list built in the software, and
provided (configured) for a user to enter personal basic data, an intake food list and an exercise record, such that the user can enter personal information including latest food intake, exercise, medication, professional medical advice, medical history, heart rate, blood pressure, weight, body fat, and sleep condition anytime through the support of cable and wireless networks, wherein
the built-in software provides a recommended food list suitable for the user.

2. The physiological condition, diet and exercise plan recommendation and management system as recited in claim 1, further comprising an exercise planning program installed in the software, such that after the software receives necessary information including the user's personal updated physiological data, food list and exercise record, a copy of a recommended food list and a copy of a planned and recommended exercise program are listed.
